(19)

Europäisches
Patentamt
European
Patent Office
Office européen
des brevets

(11) **EP 1 820 447 B1**

(12) **EUROPEAN PATENT SPECIFICATION**

(45) Date of publication and mention
of the grant of the patent:
**06.06.2012 Bulletin 2012/23**

(51) Int Cl.:
*G06T 5/40* (2006.01)       *G06T 1/00* (2006.01)
*A61B 6/00* (2006.01)

(21) Application number: **05811692.2**

(86) International application number:
**PCT/JP2005/021949**

(22) Date of filing: **30.11.2005**

(87) International publication number:
**WO 2006/062013 (15.06.2006 Gazette 2006/24)**

(54) **IMAGE PROCESSING DEVICE, IMAGE PROCESSING METHOD, AND IMAGE PROCESSING PROGRAM**

BILDBEARBEITUNGSVORRICHTUNG, BILDBEARBEITUNGSVERFAHREN UND
BILDBEARBEITUNGSPROGRAMM

DISPOSITIF, PROCEDE ET PROGRAMME DE TRAITEMENT D'IMAGE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **10.12.2004   JP 2004358480**

(43) Date of publication of application:
**22.08.2007   Bulletin 2007/34**

(73) Proprietor: **Konica Minolta Medical & Graphic, Inc.
Hino-shi,
Tokyo 191-8511 (JP)**

(72) Inventor: **KAJI, Daisuke
Hino-shi, Tokyo 191-8511 (JP)**

(74) Representative: **Gille Hrabal
Patentanwälte
Brucknerstrasse 20
40593 Düsseldorf (DE)**

(56) References cited:
**EP-A- 1 248 234        JP-A- 2001 120 524
JP-A- 2003 337 942      US-A1- 2001 038 707
US-A1- 2002 057 827     US-A1- 2003 123 617
US-A1- 2003 160 834**

EP 1 820 447 B1

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**Description**

**TECHNICAL FILED**

The present invention relates to an image processing apparatus, image processing method and image processing program for processing a radiographic image, particularly to an image processing apparatus, image processing method and image processing program for capable of adequate image processing in a highly versatile state.

**BACKGROUND**

In recent years, the digitization of an image and enhancement of the definition is making rapid progress in the field of the radiographic imaging apparatus that converts the transmission X-ray through a test subject into an image data. In this field of technology, the image data obtained from the transmitted X-ray includes a rich variety of information of the disease or lesion of the subject.

When such image data is shown on various types of display apparatuses and diagnosis is performed by operator inspection, means must be provided to ensure that a large proportion of information of the disease or lesion of the subject can be easily captured by human vision.

In this case, the image data is displayed after having been transformed with the gradation transformation characteristic by a gradation transformation processing section provided on the image processing apparatus in such a way that the region of interest can be easily captured by human vision.

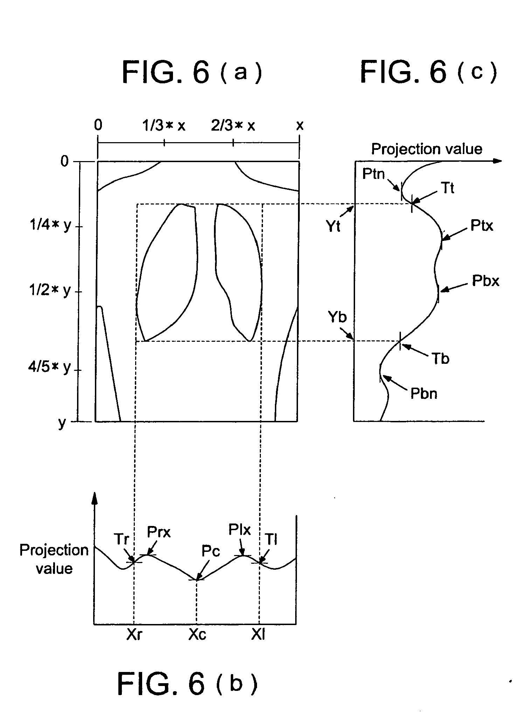

The following describes the image processing method commonly practiced in a prior art, referring to Fig. 6, using an example of the method for applying gradation transformation to the image data for the front view of the chest. Fig. 6 (a) shows the image data of the front view of the chest excluding the portion outside the irradiation field. Fig. 6 (b) shows the projection in the x-axis direction of the image shown in Fig. 6 (a). Fig. 6 (c) shows the projection in the y-axis direction of the image shown in Fig. 6 (a). The region of interest in the thorax of the front view of the chest is set in the following Steps S01 through S05, and based on the region of interest having been set (hereinafter referred to as "ROI (Region of Interest)", the gradation transformation characteristic is determined to perform gradation transformation.

Step S01: To Obtain the projection value (cumulative value in one direction of the numerical data, such as density values, luminance values, etc. of the image) in the vertical direction (y-axis direction in Fig. 6 (a)) of the image data excluding the upper and lower portions and the portion outside the irradiation field which have little effect on the entire image (Fig. 6 (b)).

Step S02: To assign the point where the projection has the minimum value (Pc) within the range of one third of the center (1/3*x through 2/3*x) in Fig. 6 (a)) from the projection in the vertical direction having been obtained to the column (Xc) of the median.

Step S03: To find out the points where the projection values are equal to or smaller than the threshold values (T1, Tr), from the columns (2/3*x, 1/3*x in Fig. 6 (a)) of one thirds of the entire image on the right and left toward the outside (in the right/left direction) of each image. Then, to assign the first points where the projection values are equal to or smaller than the threshold value (T1, Tr), to the left/right ends (X1, Xr), respectively. In this case, the threshold values (T1, Tr) are calculated from the following formulas, based on the minimum value Pc of the projection value within the range of 1/3 at the center, and the maximum values (P1x, Prx) of the projection values from the column of one thirds of the entire image.

$$Tl = ((k1 - 1) *Plx + Pc)/k1$$

$$Tr = ((k2 - 1) *Prx + Pc)/k2$$

where k1 and k2 are constants.

Step S04: To obtain the projection in the horizontal direction (x-axis direction in Fig. 6 (a)) in the region enclosed by the left and right ends determined in the previous Step (Fig. 6 (c)).

Step S05: To find out the point where the projection values in the horizontal direction are equal to or smaller than threshold values (Tt, Tb), from the lines of quarter and half (1/4*y, 1/2*y in Fig. 6) of the entire image respectively to the upper and lower directions toward the outside (in the vertical direction) of each image. Then, to assign the first points where the projection values are equal to or smaller than the threshold value (Tt, Tb), to the top and bottom ends (Yt, Yb) of the right lug field, respectively.

The threshold values Tt, Tb are respectively calculated according to the following formulas based on:

the maximum value Ptx of the projection values within the range of 1/4*y through 1/2*y of the entire image:

the maximum value Pbx of the projection values within the range of 1/2*y through 4/5*y of the entire image;
the minimum value Ptn of the projection values within the range in an upward direction of the image from the line of the maximum value; and
the minimum value Pbn of the projection values within the range in a downward direction of the image from the line Pbx.

$$Tt = ((k3 - 1) *Ptx + Ptn)/k3$$

$$Tb = ((k4 - 1) *Pbx + Pbn)/k4$$

where k3 and k4 are constants.

The range of the region of interest to be recognized can be adjusted by changing the parameters k1 through k4 utilized to find the threshold values in the aforementioned formulas.

The setting of the ROI is not limited to the case of setting by analyzing the image profile, as described above. For example, as disclosed in the Unexamined Japanese Patent Application Publication No. H5-7578, the image data of each pixel is compared with the threshold value determined by the discriminant analysis method or the like. Based on the result of comparison, an identification symbol is added for each pixel. Labeling is provided for each pixel group exhibiting a continuation of the identification symbols, which indicate that the value is equal to or greater than the threshold value, and the lung field region is extracted. Based on the lung field region having been extracted, it is possible to set the ROI so as to include the lung field and the lower region of the diaphragm.

As disclosed in the Unexamined Japanese Patent Application Publication No. S62-26047 (Tokkaisho), the lung field is identified by detection of the outline of the lung field according to the boundary point tracing method. Based on the lung field having been identified, it is possible to set the ROI so as to include the lung field and the lower region of the diaphragm.

Further, when photographing is performed, the most important portion for diagnosis is generally set at the center of the irradiation field. Accordingly, it is also possible to make such arrangements that a circular or rectangular region is arranged at the center of the region within the irradiation field, to set the ROI.

Representative values D1 and D2 are set according to the cumulative histogram of the image data inside the ROI having been set. Then the representative values D1 and D2 are set as the levels of image data wherein the cumulative histogram exhibits a predetermined ratio of m1 and m2.

When the representative values D1 and D2 have been set, reference is made to a predetermined normalization processing lookup table, normalization processing is carried out, wherein the levels of the representative values D1 and D2 are transformed into desired reference signal values T1 and T2, as shown in Fig. 8. In this case, the characteristic curve CC indicates the level of the signal outputted, according to the dose of the radiation applied to the subject.

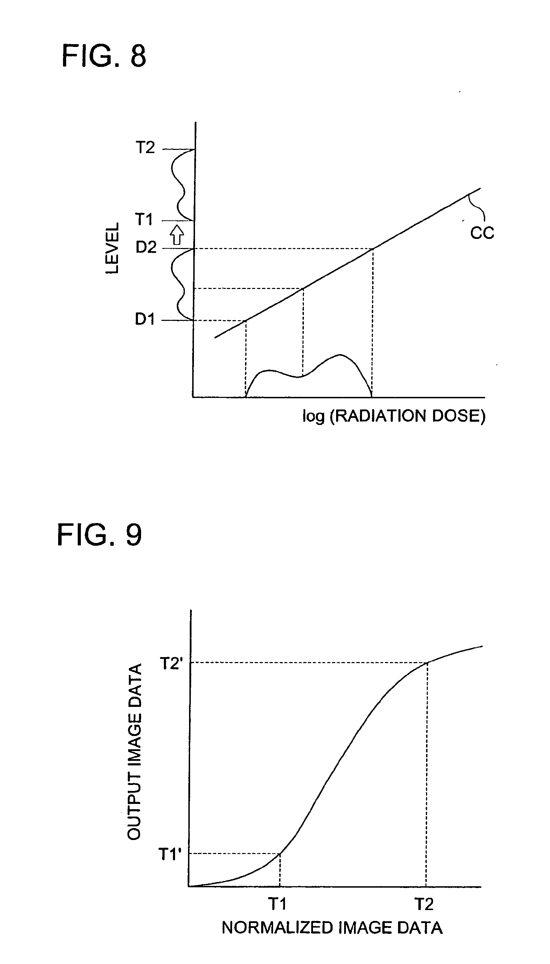

Then gradation transformation processing is applied to the normalized image data obtained by normalization processing. The gradation transformation characteristic shown in Fig. 9 is used in for gradation transformation processing, whereby the parameter values of the reference signal values T1 and T2 of the normalized image data are transformed into the levels T1' and T2'. These levels T1' and T2' correspond to the predetermined luminance or photographic density of the output image.

Here the gradation transformation characteristics are stored in the memory in the LUT (Look-Up Table) format, and the parameters of the gradation transformation characteristic, e.g., shift value (S) or gradient (value G), are set and adjusted, corresponding to the photographed site, photographing conditions, and photographing method. After the gradation transformation characteristic has been optimized, they are used for gradation transformation of the image data. Such a technique is disclosed, for example, in the Patent Document 1.

To explain the index for showing the result of image processing, it is described about a value S in the gradation transformation characteristic in the Patent Document 2 given below.

The Patent Document 3 discloses a technique of improving the sharpness of an image by the frequency enhancement processing applied to the image data having been subjected to gradation transformation:

Patent Document 1: Unexamined Japanese Patent Application Publication No. H09-16762 (page 1, Fig. 1)
Patent Document 2: Unexamined Japanese Patent Application Publication No. 2002-133410 (page 1, Fig. 1)
Patent Document 3: Unexamined Japanese Patent Application Publication No. 2001-120524

US 2001/0038707 discloses a system for improving the visibility of digital radiographic images. A region of interest is identified. Gradation conversion is applied. The gradation gradient depends on the acquired data. Subsequently, frequency enhancement processing is applied, depending is well on the input data. Finally, dynamic range compression is applied, to obtain the output image.

However, not a single of the mentioned documents do provide disclosure about the image processing condition calculation section calculates said gradation transformation characteristic curve having a gradient G smaller than a gradient obtained by photographing with a screen film system, and calculates said frequency enhancement characteristic curve for frequency enhancement from a low frequency region in which spatial frequency is lower than 0.5 cycle/mm, based on said gradation transformation characteristic curve.

## DISCLOSURE OF THE INVENTION

## PROBLEM TO BE SOLVED BY THE INVENTION

The image data obtained by photographing a subject has different characteristic and condition for each site. This requires individual determination processing for each site. To provide appropriate image processing for each site, various setting is required to the operator. To be specific, the characteristics and conditions are different depending on respective sites of a subject, and the operator's setting is necessary for image processing. This involves a problem of limited versatility in image processing.

For example, when gradation transformation processing and frequency enhancement processing are performed, a desired contrast can be obtained by changing the gradation transformation characteristic or by changing the frequency enhancement characteristic. In the image visibility range, the visibility range can be expanded by making the gradation transformation characteristic to be lower than the average gradient, or by equalization processing.

As described above, when adjustment by a plurality of parameters is required for one object, the operator actually outputted images to make comparison. Thus, the final parameters were determined according to the operator's decision.

In a method in a prior art, the parameters are determined with factors of the algorithm or programming for processing. This makes intuitive understanding very difficult.

For example, as described above, the image contrast can be adjusted by the method for changing the $\gamma$ value representing the average gradient of the LUT by gradation transformation processing.

In the meantime, frequency enhancement processing can be considered to provide a method of adjusting the image sharpness. However, the image contrast is affected by frequency enhancement processing. Especially when the enhancement from the low-frequency component is carried out, the response of the larger image components is manipulated. This will have a serious impact on image contrast.

Thus, if the operator has applied frequency enhancement processing to adjust the sharpness after adjusting the image contrast using the $\gamma$ value, the image contrast will be affected, and two types of processings must be adjusted before desired image processing parameters are obtained.

Because of this problem, when the operator does not know the details of image processing, changing to appropriate values has been very difficult. When a plurality of parameters were dependent on each other, and adjustments were made between them for appropriate values to get the optimum parameters, the appropriate values could hardly be obtained without sufficient knowledge about the specification of image processing.

Further, the aforementioned parameters are determined according to different factors for each site to be photographed. For example, when ribs are photographed, there are various forms in positioning. An image includes a large lung field in some cases. In other cases, a large proportion of the image is occupied by the portion below the diaphragm. As described above, one and the same site photographed contains a great variety of density distribution patterns. In the phase of diagnosis, appropriate processing must be applied, corresponding to the structure of the image.

To be specific, the images are different, depending on the photographed site. Appropriate parameters cannot be ensured without sufficient knowledge on the aforementioned specifications of image processing for the respective photographed sites. Thus, it has been almost impossible to achieve appropriate image processing.

For the reasons mentioned above, the aforementioned prior technique involves a problem of low gradation stability of the output image. To be specific, when the gradation transformation characteristic is set to get the density so that the output image can be easily captured by human vision, and the image data is subjected to gradation transformation based on the gradation transformation characteristic, the density of the output image is greatly deviated from the desired level, if there is a slightest calculation error contained in the values used for gradation transformation of the representative values D1 and D2 or reference signal values T1 and T2. In such a case, when the outputted image is verified and the subject is diagnosed, there is concern about the possibility of causing such a serious problem that the disease or lesion of the subject will be overlooked.

An object of the present invention is to solve problems as described above and to provide an image processing apparatus, image processing method and image processing program capable of appropriate image processing in a highly versatile state regardless of the site of a test subject or the setting by an operator.

## MEANS FOR SOLVING PROBLEMS

The problems described above are solved by the invention in accordance with claim 1.

Further embodiments of the invention are according to claims 2-14. For practicability, the claims use the expression "characteristic curve" while the abbreviated term "characteristic" has been maintained in the description

## EFFECTS OF THE INVENTION.

As has been described above, the following effects are attained in accordance with the invention.

(1) In accordance with claim 1 in the invention, gradation transformation processing is performed on image data obtained by photographing a subject, based on a gradation transformation characteristic having a predetermined gradient G, and frequency enhancement processing is performed on the image data having been subjected to the gradation transformation processing, based on a frequency enhancement characteristic being a characteristic of enhancement degrees for respective frequencies. Herein, a gradation transformation characteristic is calculated, the gradation transformation characteristic having a gradient G smaller than a gradient obtained by photographing with a screen film system, and a frequency enhancement characteristic is calculated to perform frequency enhancement from a low frequency region in which spatial frequency is smaller than 0.5 cycle/mm, based on the gradation transformation characteristic.

In this case, the fluctuation in gradation is reduced by the process of gradation transformation according to the gradation transformation characteristic having a gradient G smaller than the gradient obtained by photographing with a screen film system. The contrast of each portion of the image is achieved by the process of frequency enhancement according to the frequency enhancement characteristic wherein frequency enhancement is carried out from the low-frequency region with a spatial frequency below 0.5 cycle/mm.

As a result, the fluctuation in gradation is reduced regardless of the site of a test subject or the setting by the operator. A sufficient contrast is obtained in each part of the image. This arrangement provides appropriate image processing in a highly versatile state.

(2) In accordance with claim 2 in the invention, the gradation transformation characteristic calculated in above (1) is compared with a standard image processing condition calculated based on a characteristic amount calculated from image data. Thus, it is determined whether the gradation transformation characteristic calculated in above (1) is within an appropriate range. When it is determined that this gradation transformation characteristic is not within the appropriate range, the condition is changed and the gradation transformation characteristic in above (1) is recalculated.

Herein, the appropriate range is set, using a value indicating a preset appropriate range or a value indicating an appropriate range inputted from a scan-input section or the like.

As a result, appropriate gradation transformation characteristic can be calculated, thereby the fluctuation in gradation is reduced regardless of the site of a test subject or the setting by the operator, and a sufficient contrast is obtained in each part of the image. This arrangement provides appropriate image processing in a highly versatile state.

(3) In accordance with claim 3 in the invention, a region detection section is provided, and the quantity of pixels outside the irradiation field region or inside the direct radiation region detected by the region detection section is increased. Thus, the image processing condition calculation section changes the condition to recalculate the gradation transformation characteristic.

As a result, image processing conditions can be calculated according to the appropriate range, thereby the fluctuation in gradation is reduced, regardless of the site of a test subject or the setting by the operator, and a sufficient contrast is obtained in each part of the image. This arrangement provides appropriate image processing in a highly versatile state.

(4) In accordance with claim 4 in the invention, when the image processing condition calculation section calculates a gradient G such as to be smaller than a gradient obtained by photographing with a screen film system, an image processing condition is calculated such that, with respect to pixels in a predetermined region and difference in signal value between which is greater or equal to 1 before gradation transformation processing, the difference in signal value does not become zero after the gradation transformation processing.

As a result, a state is maintained where contrast is not lost even among the pixels with little difference in the signal value, thereby the fluctuation in gradation is reduced, regardless of the site of a test subject or the setting by the operator, and a sufficient contrast is obtained in each part of the image. This arrangement provides appropriate image processing in a highly versatile state.

(5) In accordance with claim 5 in the invention, in the frequency enhancement processing that makes enhancement from a low frequency region in which spatial frequency is lower than 0.5 cycle/mm, an image processing condition is calculated such that one of an average, maximum, and minimum values of contrast in a predetermined region is constant.

As a result, the statistical properties after image processing in a predetermined region can be maintained to be unchanged, thereby the fluctuation in gradation is reduced, regardless of the site of a test subject or the setting by the operator, and a sufficient contrast is obtained in each part of the image. This arrangement provides appropriate image processing in a highly versatile state.

(6) In accordance with claim 6 in the invention, equalization processing is performed on image data obtained by photographing a subject such that a minimum contrast amplification factor after processing in a predetermined region becomes a predetermined value.

As a result, the minimum contrast amplification factor after the processing in a predetermined region by the process of equalization becomes a predetermined value, thereby the fluctuation in gradation is reduced, regardless of the site of a test subject or the setting by the operator, and a sufficient contrast is obtained in each part of the image. This arrangement provides appropriate image processing in a highly versatile state.

(7) In accordance with claim 7 in the invention, in a case of determining whether the gradation transformation characteristic calculated in above (1) is within an appropriate range, an appropriate range is determined for a state where, with respect to pixels in a predetermined region and difference in signal value between which is greater or equal to 1 before gradation transformation processing, the difference in signal value does not become zero after the gradation transformation processing.

As a result, appropriate image processing conditions can be calculated with an assumption that appropriate range is determined to be a state where the contrast is not lost even among the pixels with small differences in the signal values, thereby the fluctuation in gradation is reduced, regardless of the site of a test subject or the setting by the operator, and a sufficient contrast is obtained in each part of the image. This arrangement provides appropriate image processing in a highly versatile state.

(8) In accordance with claim 8 in the invention, a predetermined fixed value is used as the gradient G of a gradation transformation characteristic.

As a result, the fluctuation in gradation is reduced by the fixed gradient G, regardless of the site of a test subject or the setting by the operator. Fluctuation due to image analysis is also reduced, and a sufficient contrast is obtained in each part of the image. This arrangement provides appropriate image processing in a highly versatile state.

(9) In accordance with claim 9 in the invention, the predetermined region in above (4) to (7) is detected, according to a predetermined reference that is based on one of a predetermined histogram ratio, setting of ROI, and analysis result of a characteristic amount.

As a result, the image processing conditions are determined based on the diagnostically important region, various forms of fluctuation are reduced, and a sufficient contrast is obtained in each part of the image. This arrangement provides appropriate image processing in a highly versatile state.

(10) In accordance with claim 10 in the invention, there is provided an operation section via which input related to setting or change of gradation transformation characteristic is made, wherein the image processing condition calculation section determines the gradation transformation characteristic, referring to input via the operation section.

As a result, image processing conditions are determined in a state where the will of the operator is reflected, various forms of fluctuation are reduced, and a sufficient contrast is obtained in each part of the image. This arrangement provides appropriate image processing in a highly versatile state.

(11) In accordance with claim 11 in the invention, gradation transformation processing is performed on image data obtained by photographing a subject, based on a gradation transformation characteristic having a gradient G smaller than a gradient obtained by photographing with a screen film system; a frequency enhancement characteristic is calculated to perform frequency enhancement from a low frequency region in which a spatial frequency is lower than 0.5 cycle/mm, based on the gradation transformation characteristic; and

frequency enhancement processing is performed on the image data having been subjected to the gradation transformation processing, based on the calculated frequency enhancement characteristic.

As a result, the fluctuation in gradation is reduced, regardless of the site of a test subject or the setting by the operator, and a sufficient contrast is obtained in each part of the image. This arrangement provides appropriate image processing in a highly versatile state.

(12) In accordance with claim 12 in the invention, a standard image processing condition, which is calculated based on a characteristic amount calculated from image data, and the gradation transformation characteristic of above (11) are compared. Thus, it is determined whether or not the gradation transformation characteristic of above (11) is within an appropriate range. When it is determined that the gradation transformation characteristic is not within the appropriate range, the gradation transformation characteristic of above (11) is recalculated with a change of a condition of calculation.

As a result, appropriate gradation transformation characteristic can be calculated, thereby the fluctuation in gradation is reduced, regardless of the site of a test subject or the setting by the operator, and a sufficient contrast is obtained in each part of the image. This arrangement provides appropriate image processing in a highly versatile state.

(13) In accordance with claim 13 in the invention, gradation transformation processing is performed on image data obtained by photographing a subject, based on a gradation transformation characteristic having a gradient G smaller than a gradient obtained by photographing with a screen film system; a frequency enhancement characteristic is calculated to perform frequency enhancement from a lower frequency region in which a spatial frequency is lower than 0.5 cycle/mm, based on the gradation characteristic; and frequency enhancement processing is performed on the image data having been subjected to the gradation transformation processing, based on the frequency enhancement characteristic calculated by the calculation processing.

Thus, the fluctuation in gradation is reduced, regardless of the site of a test subject or the setting by the operator, and a sufficient contrast is obtained in each part of the image. This arrangement provides appropriate image processing in a highly versatile state.

(14) In accordance with claim 14 in the invention, a standard image processing condition, which is calculated based on a characteristic amount calculated from image data, and the gradation transformation characteristic of above (13) are compared. Thus, it is determined whether or not the gradation transformation characteristic in above 813) is within an appropriate range. When it is determined that the gradation transformation characteristic is not within the appropriate range, the gradation transformation characteristic in above (13) is recalculated with a change of a calculating condition.

As a result, appropriate gradation transformation characteristic can be calculated, thereby the fluctuation in gradation is reduced, regardless of the site of a test subject or the setting by the operator, and a sufficient contrast is obtained in each part of the image. This arrangement provides appropriate image processing in a highly versatile state.

## BRIEF DESCRIPTION OF THE DRAWINGS

Fig. 1 is a block diagram showing the entire structure or a flow of the entire processing in an embodiment of the present invention;

Fig. 2 is a flow chart showing an example of processing in the embodiment of the present invention;

Fig. 3 is a characteristic figure representing the characteristic of gradation transformation processing and frequency enhancement processing in the embodiment of the present invention;

Fig. 4 is a block diagram representing the entire structure or a flow of the entire processing in the embodiment of the present invention;

Fig. 5 is another block diagram representing the entire structure or a flow of the entire processing in the embodiment of the present invention;

Fig. 6 is an explanatory diagram showing how an image is processed on the pectoral region;

Fig. 7 is an explanatory diagram showing the value G and value S in the embodiment of the present invention;

Fig. 8 is an explanatory diagram of normalization processing; and

Fig. 9 is an explanatory diagram showing gradation transformation characteristic.

## DESCRIPTION OF REFERENCE NUMERALS

100 Image processing apparatus
110 Image data input section
120 Standard image processing condition calculation section
130 Image processing condition calculation section
140 Image processing condition determining section
160 Image processing section
180 Image output section

**BEST MODE FOR PRACTICING THE INVENTION**

Referring to the drawings, the following describes the best mode for practicing the present invention:

The following describes preferred embodiments of image processing apparatus in the best mode for practicing the invention. It is to be understood, however, that the present invention is not limited thereto.

(1st EMBODIMENT)

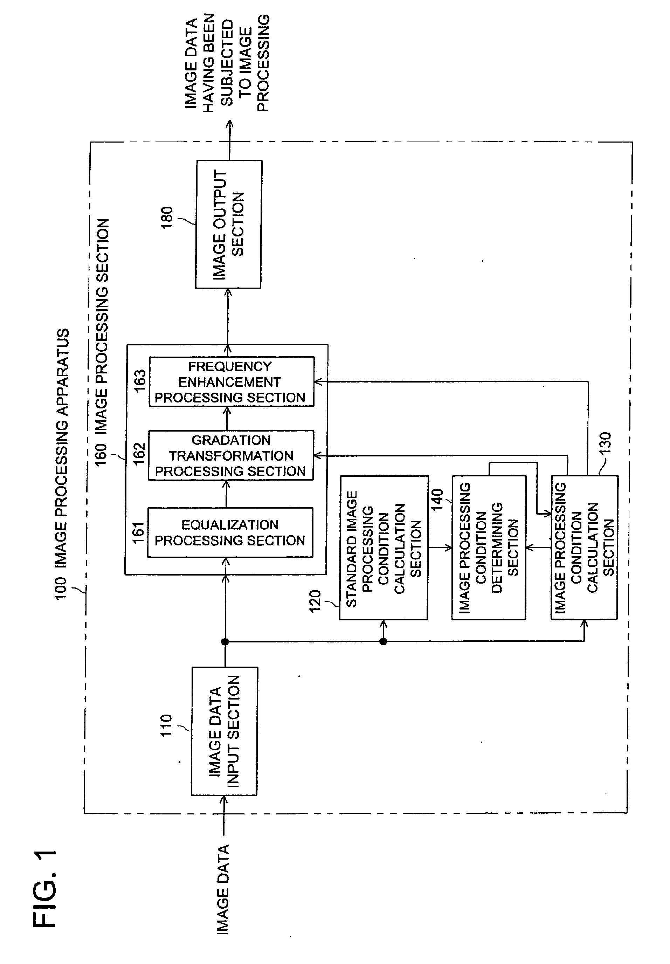

A first embodiment can be implemented by hardware, firmware or software. Fig. 1 is a function block diagram according to the procedures of the respective steps, means, and routines.

Entire structure:

The image processing apparatus 100, shown in Fig. 1, applies various image processing, such as equalization, gradation transformation and frequency enhancement processing on image data obtained by photographing a subject, based on an index, corresponding to the image data.
Equalization processing is image processing that compresses the dynamic range of an image signal, based on the a non-sharp image signal so that even an image of a wide dynamic range is set within the density range that allows easy viewing of the entire image.
Gradation transformation processing performs gradation transformation, based on a predetermined LUT having gradation transformation characteristic, thereby generating an image of a desired gradation (output signal value). Herein, the image data is subjected to gradation transformation with the gradation transformation characteristic of the gradation transformation processing section of the image processing apparatus, and is displayed such that the region of interest in particular can be easily captured by human vision. The aforementioned gradation transformation characteristics stored in a LUT format are stored in a memory. The parameters of the gradation transformation characteristic, e.g., shift value (value S) and gradient (value G) are set and adjusted, as necessary. After having been optimized, the gradation transformation characteristic is used for gradation transformation of image data.
Frequency enhancement processing achieves a sharper image of the structure of a human body contained in the image data having been photographed, by controlling the spatial frequency characteristic of an image.
As shown in Fig. 1, the image processing apparatus 100 includes:

an image data input section 110 via which image data is inputted from the outside;
a standard image processing condition calculation section 120 for calculating standard image processing condition based on the characteristic amount extracted from the image data;
an image processing condition calculation section 130 for calculating image processing conditions that are specific in the present embodiment;
an image processing condition determining section 140 (determining section of the present invention) for determining, based on the standard processing conditions to determine whether or not the image processing conditions specific in the present embodiment are within an appropriate range;
an image processing section 160 for execution of equalization processing, gradation transformation processing and frequency enhancement processing; and
an image output section 180 for outputting the image data having been subjected to image processing, to the outside.

The image processing section 160 includes at least an equalization processing section 161 for execution of equalization processing, a gradation transformation processing section 162 for execution of gradation transformation processing and a frequency enhancement processing section 163 for execution of frequency enhancement processing.
Respective sections (respective means), shown in Fig. 1 and described above, are components of the image processing apparatus 100. They also constitute the respective steps of an image processing method and the respective routines of an image processing program. Further, the image processing apparatus 100 can be structured with a combination of a CPU, memory and processing programs, and can also be structured, employing a programmable gate array and others.

Process flow:

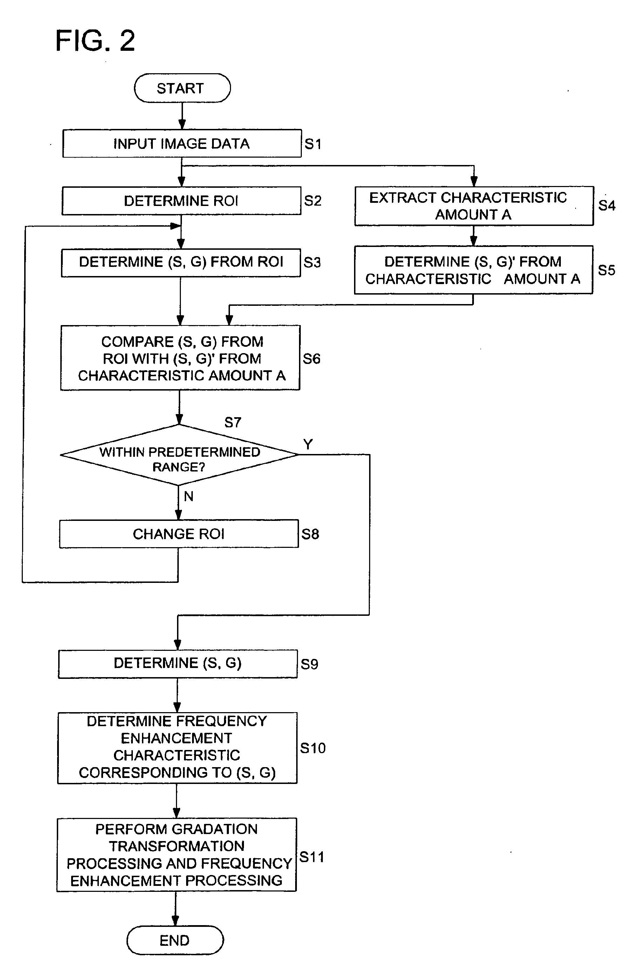

Referring to the flowchart in Fig. 2, the following describes the operation (processing) state of the image processing apparatus 100 in the present embodiment.

(a) Acquisition of image data:

Regarding a medical image obtained by radiographic photographing or other, image data input section 110 acquires image data transmitted from a radiographic image photographing device or radiographic image reading device (S1 in Fig. 2).

To be specific, the image data input section 110 used in the image data input step, for example, detects radiation passing through the subject, and generates an image data. Alternatively, the image data input section 110 receives the image data generated from the radiation passing through the subject, from an external device.

As a concrete example, one using stimulable phosphor plate is disclosed in the Unexamined Japanese Patent, Application Publication No. H11-142998 and Unexamined Japanese Patent Application Publication No. 2002-156716. As those using a flat panel detector (FPD) as an input apparatus, one is disclosed in the Unexamined Japanese Patent Application Publication No. H6-342098, wherein detected X-rays are directly converted into an electric charge and are obtained as image data, and another one is disclosed in the Unexamined Japanese Patent Application Publication No. H9-90048, wherein the detected X-rays are converted into light, which is received and is converted into an electric charge indirectly.

(b) Calculation of image processing conditions:

As calculations of image processing conditions, there are calculations of gradation transformation characteristic and that of frequency enhancement characteristic.

(b-1) Calculation of gradation transformation characteristic:

The image data acquired by the image data input section 110 is sent to the image processing section 160 for image processing. Further, it is also sent to the standard image processing condition calculation section 120 and image processing condition calculation section 130 so that image processing conditions are calculated.

In the image processing condition calculation section 130, the region of interest (ROI) is recognized and determined by a built-in a region-of-interest detecting section (not shown) (S2 in Fig. 2). The image processing condition calculation section 130 analyzes the distribution and the like of the image data in the region of interest having been recognized, whereby the gradation transformation characteristic is calculated (S3 in Fig. 2).

Herein, when frequency enhancement processing is executed in the frequency enhancement processing section 163 based on the frequency enhancement characteristic (described later), the image processing condition calculation section 130 calculates the gradation transformation characteristic wherein the gradient G in the gradation transformation processing is reduced to allow execution of frequency enhancement processing that makes enhancement from the low frequency region.

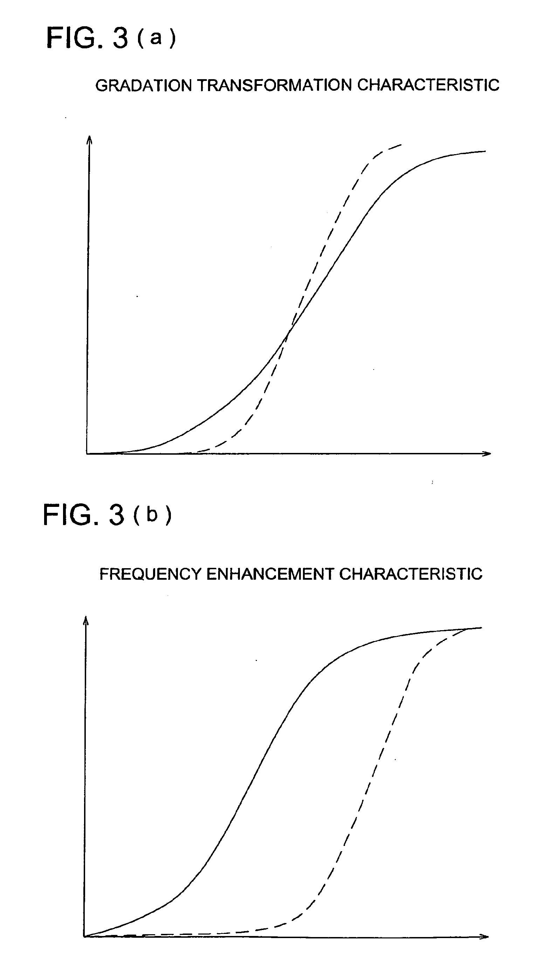

Fig. 3 (a) is a characteristic figure showing the comparison between the gradient (dashed line) of the gradation transformation characteristic in a prior art and the gradient (solid line) of the gradation transformation characteristic in the present invention. In this characteristic figure, the radiation dose is plotted along the horizontal axis, while density is plotted along the vertical axis.

The greater the gradient G of the gradation transformation characteristic in this gradation transformation processing, the higher the contrast of the image data having been subjected to gradation transformation processing. In a prior art, gradation transformation processing is conducted according to the gradation transformation characteristic having a greater gradient G so that higher contrast will be obtained for the region important for diagnosis. In this method, however, a fluctuation in the gradation transformation processing is increased by the deviations of the image processing conditions, as a problem in return for higher contrast.

In the present embodiment, the gradient G of the gradation transformation characteristic is made smaller in the process of gradation transformation processing, and the entire image data is provided with some contrast. Strong frequency enhancement processing is applied from a low-frequency region in the subsequent enhancement processing, whereby a high contrast is given to the human structure.

When the gradient G is set to a low value in this way, the image processing condition calculation accuracy is allowed to have a tolerance range, and a high degree of gradation stability is achieved. Thus, image processing condition calculation accuracy is alleviated. This makes it possible to reduce or eliminate the need of classification in the ROI calculation based on the site setting key operation on the console used in a prior art. This arrangement, therefore, results in a substantial reduction in the key operation on the console or elimination of the key operation.

For the gradation transformation characteristic of the gradient G obtained in this manner, desired transformation characteristic can be provided by shifting the gradation transformation characteristic curve. The amount of this shift is defined as a value S. For example, while the value G as a normal gradation transformation characteristic is .about 3.0, the value G is set to about 1.5 through 2.0 in the present embodiment. That is, the gradient G is set to about 1/2 through 2/3 of the gradient obtained through the photographing by a normal screen film (S/F) system.

(b-2) Image processing condition appropriateness:

Parallel with the determination of the ROI and the calculation of the values S and G of the gradation transformation characteristic, based on the ROI, as described above, the standard image processing condition calculation section 120 extracts the characteristic amount of A from the image data (S4 in Fig. 2). Based on the characteristic amount A, the values S and G are calculated in the same manner. The result is represented by (S, G)' (S5 in Fig. 2).

The (S, G)' obtained by the standard image processing condition calculation section 120 and the (S, G) in the present embodiment obtained by the image processing condition calculation section 130 are compared by the image processing condition determining section 140 (S6 in Fig. 2), and it is determined whether or not the aforementioned (S, G) is within a predetermined range from the aforementioned (S, G)'.

When the aforementioned (S, G) is not within the predetermined range from the aforementioned (S, G)' (N in S7 in Fig. 2), the image processing condition determining section 140 notifies error determination to the image processing condition calculation section 130.

By the image processing condition calculation section 130 having received the notification of this error determination, the region of interest (ROI) is recognized again (S8 in Fig. 2). In this case, the image processing condition calculation section 130 changes the region of interest so as to increase the number of the pixels outside the irradiation field region detected by the region detecting section (not shown) or directly in the radiation region. The image processing condition calculation section 130 changes the conditions, so that the image processing conditions are again calculated (S3 in Fig. 2).

When the aforementioned (S, G) is within the predetermined range from the aforementioned (S, G)' (Y in S7 of Fig. 2), the image processing condition determining section 140 notifies positive determination to the image processing condition calculation section 130. The image processing condition calculation section 130 having received the notification of the positive determination assigns the (S, G) at the moment to the characteristic of gradation transformation processing to be performed by the gradation transformation processing section 162 (S9 in Fig. 2).

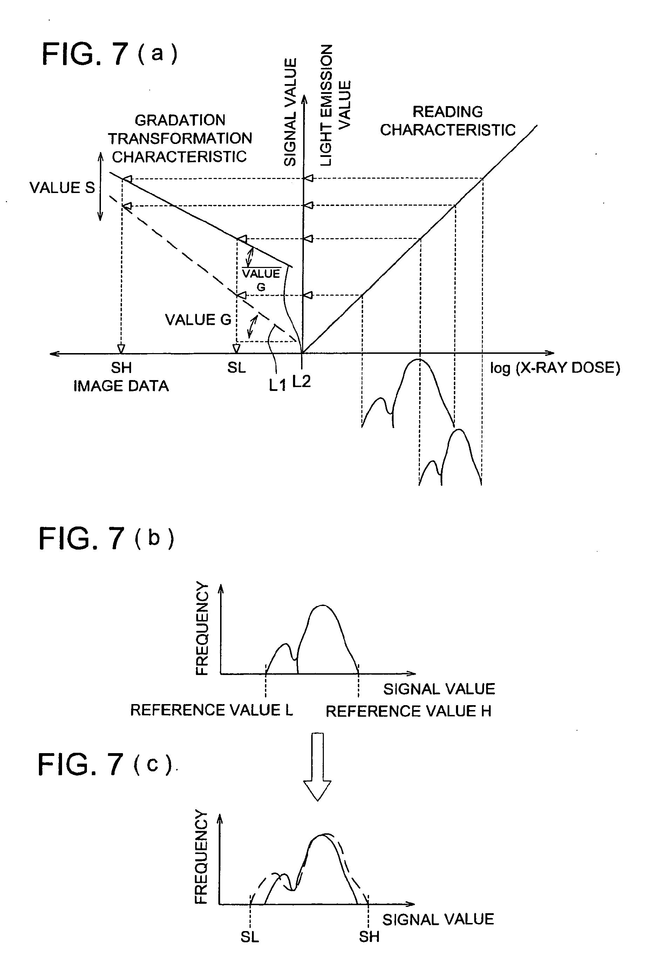

The following describes the values S and G of the gradation transformation characteristic with reference to the characteristic figure shown in Fig. 7 (a).

Fig. 7 (a) shows the different gradation transformation characteristics L1 and L2. In the quadrant wherein the L1 and L2 are shown, the input signal value is indicated by the vertical axis, and the signal value of the output image data is indicated by the horizontal axis. When the value G (gradient of the gradation transformation characteristic) is changed, the range of the signal value of the image data is also changed. This allows changing the contrast of the image. This operation is called "rotation of LUT".

When the value S (segment y of gradation transformation characteristic) is changed, the signal value of the image data is increased or decreased. This is called "shifting of LUT".

To be specific, when the aforementioned values G and S are changed, for example, the image data having the histogram (a graph showing the frequency distribution of density levels) shown in Fig. 7 (b) can be transformed into the image data containing the histogram shown in Fig. 7 (c). Then the inputted signal value can be changed to a desired density (signal value), and can be outputted.

The following describes the values S and G (S, G)' based on the characteristic amount A:

The characteristic amount A is obtained by calculating the absolute value of the edge component obtained by execution of filtering processing for extracting the high frequency range such as a differential filter or Laplacian filter. In addition, the average image density, the dispersion value of the pixel signal value in the image, representative value or the combination thereof can be used.

The values S and G (S, G)' based on the characteristic amount A is calculated to find out the values S and G in such a way that E (S', G') as a result of evaluation by the characteristic amount evaluation function becomes the characteristic amount A.

The characteristic amount evaluation function E is shown, for example, by the following formula:

$$\mathrm{E}\ (\mathrm{S},\ \mathrm{G})\ =\ \mathrm{EDGE}\ (\mathrm{S},\ \mathrm{G})/\mathrm{ORG\_E}\ (\mathrm{S},\ \mathrm{G})$$

where EDGE (S, G) represents the average contrast of the image having been transformed, according to the lookup table, and ORG_E (S, G) represents the average contrast of the original image.

For the details of calculating the values S and G based on the characteristic amount A, reference can be made to the description disclosed in the Unexamined Japanese Patent Application Publication No. 2005-109867.

(b-3) Calculation of frequency enhancement characteristic:

The image processing condition calculation section 130 determines the frequency enhancement characteristic for processing executed by the frequency enhancement processing section 163, based on the (S, G) of the gradation transformation characteristic having been determined (S10 in Fig. 2). In the present embodiment, the gradient G of the gradation transformation characteristic is set small in the process of gradation transformation processing so that some contrast is given to the entire image data. Strong frequency enhancement processing is applied from the low-frequency range by the subsequent frequency enhancement processing.

The frequency enhancement characteristic can be determined as follows, for example. "$\beta$" is given as a basic enhancement degree. This basic enhancement degree $\beta$ represents the contrast of the edge portion of the step edge of the step difference 10. For example, if the step difference has reached 20 as a result of frequency enhancement processing and gradation transformation processing, $\beta$ = 2.0. Herein, it is assumed that, if the step difference after transformation is represented by "X", then generally $\beta$ = X/10.

Assume that the degree of frequency enhancement f is f = $\beta 1$ - G1 ($\beta 1$ > G1) when the average gradient value G of the gradation transformation characteristic is G1, and the basic enhancement degree is $\beta 1$. Also assume that f = 0 when G1 > $\beta 1$. This arrangement allows the degree of frequency enhancement f to change, depending on the change in the gradient G. The increase rate of the step is maintained always at the basic enhancement degree $\beta 1$.

Further, in this case, the smaller the average gradient G is, the easier it is to supplement the contrast of the entire image by shifting the enhancement frequency band to enhancement from low frequency. In addition, the dose can be estimated to some extent from the amount of shift value S of the gradation transformation processing. Accordingly, when the dose is insufficient, the basic enhancement degree can be reduced to create an image of excellent granularity.

Fig. 3 (b) is a characteristic figure representing the comparison between the frequency enhancement characteristic (dashed line) of a prior art and the frequency enhancement characteristic (solid line) of the present embodiment. In this characteristic figure, the spatial frequency is indicated by the horizontal axis and the enhancement degree is indicated by the vertical axis.

Herein, in a prior art, enhancement is performed in the high frequency region of the spatial frequency (frequency characteristic with which about half (0.5 x Emax) of the maximum enhancement degree Emax is reached at 0.5 cycle/mm). In the present embodiment, a high enhancement degree is applied from the low-frequency region (frequency characteristic with which about half (0.5 x Emax) of the maximum enhancement degree Emax is reached at about 1/10 of the frequency characteristic of the aforementioned high frequency). In the present embodiment, when the fluctuation in gradation is to be prevented while G of (S, G) is fixed, the frequency enhancement characteristic is determined, based on the value S.

(c) Execution of image processing:

As described above, the gradation transformation characteristic determined by the image processing condition calculation section 130 is transmitted to the gradation transformation processing section 162. Then, gradation transformation processing is applied by the gradation transformation processing section 162 to the image data having been equalized by the equalization processing section 161. The frequency enhancement characteristic determined by the image processing condition calculation section 130 is transmitted to the frequency enhancement processing section 163. Then, frequency enhancement processing is applied by the frequency enhancement processing section 163 to the image data having been subjected to gradation transformation processing (S11 in Fig. 2). In this manner, the image data having been subjected to the equalization processing, gradation transformation processing and frequency enhancement processing is outputted to an external device from the image output section 180.

In the equalization processing section 161, equalization processing stronger than equalization in a prior art may be applied. Since the minimum contrast amplification factor after processing within a predetermined region reaches a predetermined value as a result of the equalization processing, the fluctuation in gradation is reduced, regardless of the site of a test subject or the setting by the operator, and appropriate image processing can be performed in a highly versatile state wherein a sufficient contrast is achieved in each part of the image.

Other Embodiment (1)

Appropriateness or inappropriateness can be determined from the result of gradation transformation processing, instead of determination of the gradation transformation characteristic (S4 through S7 in Fig. 2). To be specific, when calculation is made by the gradation transformation processing section 162 such that value G is smaller than the standard gradient, the image processing condition determining section 140 may determine appropriateness for a state where, with respect to pixels in a predetermined region and difference in signal value between which is greater or equal to 1 before gradation transformation processing, the difference in signal value does not become zero after the gradation transformation processing.

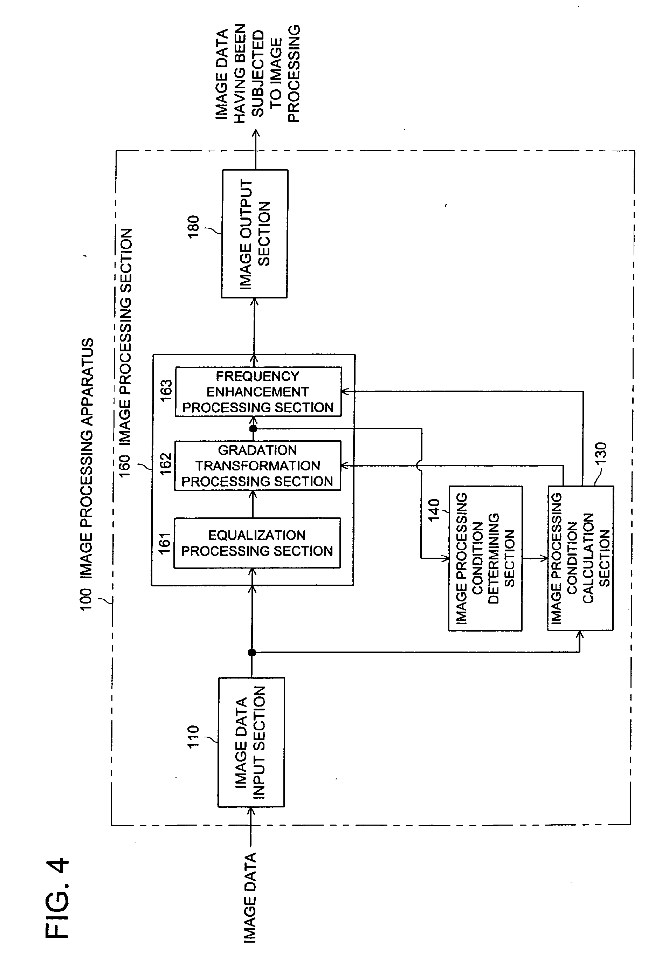

In this case, gradation transformation processing is performed by the gradation transformation processing section 162 according to the gradation transformation characteristic calculated by the image processing condition calculation section 130, as shown in Fig. 4. The result of gradation transformation processing is determined by the image processing condition determining section 140. If it is determined inappropriate, the image processing condition calculation section 130 recalculates gradation transformation characteristic upon the error notification from the image processing condition determining section 140.

In this case, it is also possible to make the following arrangements: A diagnostically important region is extracted, and if one, of the statistical values of average, maximum and minimum values of the contrast amplification factor of the region after gradation transformation processing, has exceeded the threshold value, the image processing condition determining section 140 determines inappropriateness, and issues an error notification.

As a result, appropriate image processing conditions are calculated, assuming that the appropriate range is in a state where the contrast is maintained even between pixels with a smaller difference in the signal value. Thus, the fluctuation in gradation is reduced, regardless of the site of a test subject or the setting by the operator, and appropriate image processing can be performed in a highly versatile state wherein a sufficient contrast is obtained in each part of the image.

An image edge contrast (absolute value of the pixel value) can be used for a method, the method using a characteristic amount, of recognizing the aforementioned diagnostically important region at the time of extraction thereof. Using this method allows a higher weight to be assigned to the edge portion constituting the diagnostically important structure of a subject. The edge region can be extracted by a differential filter, such as a Laplacian filter, or multiple resolution processing, such as wavelet transformation.

In addition, since the diagnostically important region is often located at the central part of the image data, it is effective to assign a greater weight to the central part of the image data. It is also effective to assign a lower weight if the density of the image is extremely high or low, or to assign a greater weight if the degree linkage with the neighboring edges is greater. Further, it is effective to examine the granularity in the vicinity of the pixels by checking the statistical amount, for example, the distribution value. If this distribution value is within a predetermined range, it is determined that the granularity is inappropriate and a lower weight is assigned.

Other Embodiment (2)

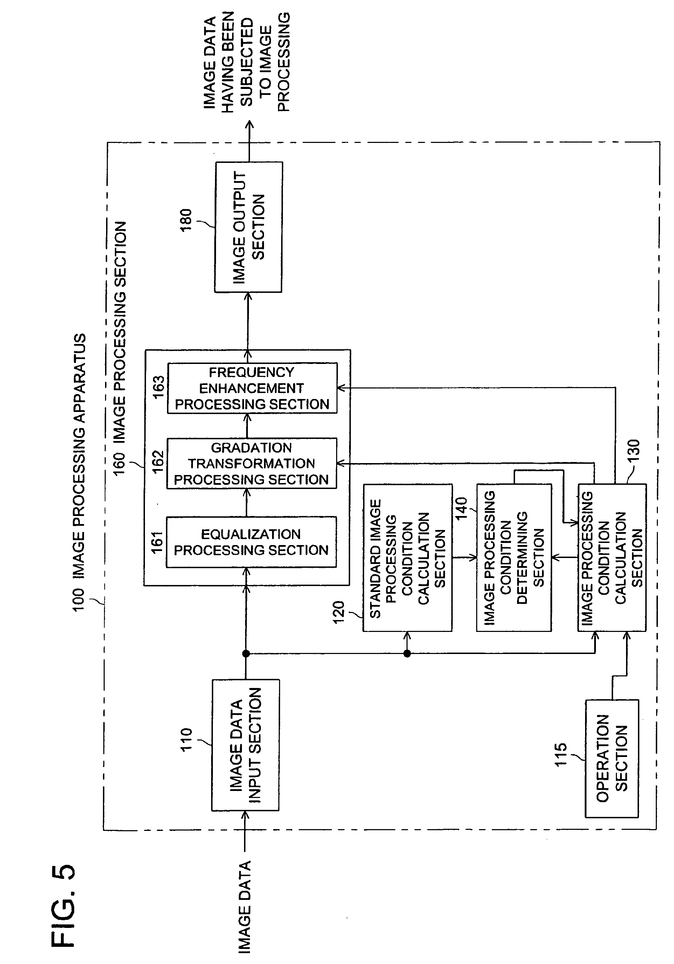

As shown in Fig. 5, the image processing apparatus 100 may incorporate an operation section 115 for receiving operation inputs by an operator. In the image processing apparatus 100 having such a structure, when a parameter on the image processing conditions is inputted via the operation section 115, the image processing condition calculation section 130 refers to the parameter inputted from the operation section 115 as well, reduces the gradient G of the gradation transformation characteristic in gradation transformation processing, to make some contrast on the entire image data. Then, image processing conditions are calculated such that strong frequency enhancement processing is executed from the low frequency region in the subsequent frequency enhancement processing. However, if inappropriateness is detected by the image processing condition determining section 140 according to the parameter inputted via the operation section 115, then the image processing conditions calculated by the image processing condition calculation section 130 is given priority over the inputted parameters.

The following describes the case of diagnosing a side of the lumbar spine by way of an example:

Generally, in the diagnosis of a side of a lumbar spine, the density of the periphery of the third vertebra is said to be set at about 1.0 in order to ensure that the output image can be easily captured by human vision. Assume, for example, that the gradient value G is set to 2.5, and the third lumbar vertebra is outputted with a density of 1.0. The signal value of the third vertebra is determined (estimated) by the histogram analysis or ROI recognition, and the shift value S is calculated such that this signal value is outputted with the density of 1.0.

To determine a reference value (reference signal value) for the signal value of the third lumbar vertebra, assume that there is a deviation of $\Delta s$ from the actual signal value of the third lumbar vertebra, and thus the output is made with a density that is deviated by $\Delta d$ from 1.0.

In the meantime, as shown in the present invention, assume that the gradient G is set to a smaller gradient than that obtained photographing with a screen film based system (Herein, gradient value G = 1.8), and contrast is applied to the image by enhancement from the low frequency region. An image can be obtained such that the structure in the image and details can be observed likewise by adjusting the enhancement degree (empirically, an output image is obtained by the enhancement degree of about 1.5 and gradient G of about 1.8, with the same density as that of a case with the enhancement degree of 0.5 and gradient G of 2.5). In this case, since the value G is 1.8/2.5 = 0.72 times, the difference in the density of the output image is 0.72 x $\Delta d$. Thus, in the present invention, the fluctuation in density can be reduced to 72 %. In other words, this eliminates the need of strict calculation (adjustment) of the shift value S. Even if some calculation error occurs, the density of the output image can be changed to 1.0.

Effects of Embodiments:

The operation of each section in the present embodiment as described above, and the effects obtained therefrom are described in the following (a) through (j):

(a) In the present embodiment, gradation transformation processing is executed on the image data obtained by photographing the subject, according to the gradation transformation characteristic containing a predetermined gradient G, and frequency enhancement processing is applied to the image data having been subjected to the aforementioned gradation transformation processing, based on a frequency enhancement characteristic, which is a characteristic regarding the enhancement degrees in respective frequencies. In this operation, the gradation transformation characteristic containing the gradient G smaller than that obtained by photographing with the screen film based system is calculated. Further, based on the aforementioned gradation transformation characteristic, the frequency enhancement characteristic is calculated for executing frequency enhancement from the low frequency region having a spatial frequency smaller than 0.5 cycle/mm.

Herein, the fluctuation in gradation is reduced by gradation transformation processing based on the gradation transformation characteristic containing the gradient G smaller than that obtained by photographing with the screen film based system, and contrast is achieved for each part of the image by frequency enhancement processing based on the frequency enhancement characteristic for frequency enhancement from the low frequency region having a spatial frequency lower than 0.5 cycle/mm.

As a result, the fluctuation in gradation is reduced, regardless of the site of a test subject or the setting by the operator, and a sufficient contrast is obtained in each part of the image. This arrangement provides appropriate image processing in a highly versatile state.

For example, when the ribs are photographed, positioning is various. A greater portion of the lung field is included in some cases, and the greater portion of the image is occupied by the site below the diaphragm in other cases. As described above, there is a great variety of density distribution for the same site to be photographed. This makes it necessary to apply appropriate processing in diagnosis, corresponding to the structure of the image. In a prior art, setting of image processing conditions by an operator has been difficult, and therefore, appropriate parameters cannot be obtained without sufficient knowledge about the details of image processing for each site to be photographed. Thus, appropriate image processing has been almost impossible. In the present embodiment, described above, however, the fluctuation in gradation is reduced, regardless of the site of a test subject or the setting by the operator, and a sufficient contrast is obtained for each part of the image. This arrangement provides appropriate image processing in a highly versatile state. Thus, a sufficient contrast is provided in all density ranges, despite a wide dynamic range for obtaining diagnostic information. This provides an image suitable for diagnosis.

(b) In the present embodiment, the gradation transformation characteristic calculated in the aforementioned (a) is compared with the standard image processing condition calculated according to the characteristic amount calculated from the image data, thereby it is determined whether or not the gradation transformation characteristic calculated in the aforementioned (1) is included in the appropriate range. If it is not within the appropriate range, the conditions are changed to recalculate the gradation transformation characteristic of the aforementioned (a). The setting of the appropriate range is made, according to the value indicating a predetermined appropriate range or the value indicating the appropriate range inputted from a scanning input section or the like.

Thus, an appropriate gradation transformation characteristic can be calculated, thereby the fluctuation in gradation is reduced, regardless of the site of a test subject or the setting by the operator, and a sufficient contrast is obtained in each part of the image. This arrangement provides appropriate image processing in a highly versatile state.

(c) In the present embodiment, a region detecting section is provided. Conditions are changed by the image processing condition calculation section such as to increase the number of the pixels outside the irradiation field region detected by the region detecting section or the pixels in the direct radiation region. Thus, gradation transformation characteristic is recalculated.

Thus, the image processing conditions can be calculated based on the appropriate region, thereby the fluctuation in gradation is reduced, regardless of the site of a test subject or the setting by the operator, and a sufficient contrast is obtained in each part of the image. This arrangement provides appropriate image processing in a highly versatile state.

(d) In the present embodiment, when calculation is made by the aforementioned image processing condition calculation section in such a way that the gradient G is smaller than that obtained by photographing with the screen film based system, image processing conditions are calculated in the following manner. That is, with respect to pixels in a predetermined region and difference in signal value between which is greater or equal to 1 before gradation transformation processing, the difference in signal value does not become zero after the gradation transformation processing.

Thus, the contrast is maintained even between the pixels with a small difference in the signal value, thereby the fluctuation in gradation is reduced, regardless of the site of a test subject or the setting by the operator, and a sufficient contrast is obtained in each part of the image. This arrangement provides appropriate image processing in a highly versatile state.

(e) In the present embodiment, when frequency enhancement processing is executed from the low frequency region having a spatial frequency lower than 0.5 cycle/mm, image processing conditions are calculated so that one of the average, maximum and minimum values will be constant.

Thus, the statistical properties after image processing in a predetermined region can be maintained constant, thereby the fluctuation in gradation is reduced, regardless of the site of a test subject or the setting by the operator, and a sufficient contrast is obtained in each part of the image. This arrangement provides appropriate image processing in a highly versatile state.

(f) In the present embodiment, equalization processing is executed on the image data obtained by photographing the subject, wherein the minimum contrast amplification factor after processing in a predetermined region becomes a predetermined value.

Since equalization processing makes the minimum contrast amplification factor after processing in a predetermined region be a predetermined value, the fluctuation in gradation is reduced, regardless of the site of a test subject or the setting by the operator, and a sufficient contrast is obtained in each part of the image. This arrangement provides appropriate image processing in a highly versatile state.

(g) In the present embodiment, the gradation transformation characteristic calculated by above item (a) is determined to be in the appropriate range in the following case. That is, with respect to pixels in a predetermined region and difference in signal value between which is greater or equal to 1 before gradation transformation processing, the difference in signal value does not become zero after the gradation transformation processing.

Thus, appropriate image processing conditions can be calculated with an assumption that appropriate range is defined by a state where the contrast is not lost even among the pixels with a small difference in the signal value, thereby the fluctuation in gradation is reduced, regardless of the site of a test subject or the setting by the operator, and a sufficient contrast is obtained in each part of the image. This arrangement provides appropriate image processing in a highly versatile state.

(h) In the present embodiment, a predetermined fixed value is used as the gradient G of gradation transformation characteristic, thereby the fluctuation in gradation is reduced, regardless of the site of a test subject or the setting by the operator, and a sufficient contrast is obtained in each part of the image. This arrangement provides appropriate image processing in a highly versatile state.

(i) In the present embodiment, the predetermined region in the aforementioned (d) through (g) is detected, according to a predetermined reference based on one of a predetermined histogram ratio, setting of the ROI and result of analyzing the characteristic amount. Thus, the image processing conditions can be determined based on the diagnostically important region, thereby considerable resistance is created against various fluctuations, and a sufficient contrast is obtained in each part of the image. This arrangement provides appropriate image processing in a highly versatile state.

(j) In the present embodiment, an operation section is provided for setting of gradation transformation characteristic or inputting of changes. The image processing condition calculation section refers to input via the operation section to determine the gradation transformation characteristic. Thus, the image processing conditions characteristic of these embodiments can be determined, with the operator's intention taken into account, thereby considerable resistance is created against various forms of fluctuation, and a sufficient contrast is obtained in each part of the image. This arrangement provides appropriate image processing in a highly versatile state.

**Claims**

1.  An image processing apparatus that performs gradation transformation processing and frequency enhancement processing on image data obtained by radiographic photographing of a subject, comprising:

    a gradation transformation processing section that performs gradation transformation processing on image data obtained by photographing a subject, based on a gradation transformation characteristics curve having a predetermined range for its gradient G;
    a frequency enhancement processing section that performs frequency enhancement processing on the image data having been subjected to the gradation transformation processing, based on a frequency enhancement characteristic curve being a characteristic curve of enhancement degrees for respective frequencies; and
    an image processing condition calculation section that calculates the gradation transformation characteristic curve for said gradation transformation processing section and the frequency enhancement characteristic curve for said frequency enhancement processing section, based on the gradation transformation characteristic curve,

wherein the image processing condition calculation section calculates said gradation transformation characteristic curve having a gradient G smaller than a gradient obtained by photographing with a screen film system, and calculates said frequency enhancement characteristic curve for frequency enhancement from a low frequency region in which spatial frequency is lower than 0.5 cycle/mm, based on said gradation transformation characteristic curve.

2. The image processing apparatus of claim 1, further comprising:

a standard image processing condition calculation section that calculates a standard image forming condition and its values G and S, based on a characteristic amount calculated from image data; and
a determining section that compares said values for G and S calculated by the standard image processing condition calculation section with the values for G and S of said gradation transformation characteristic curve calculated by the image processing condition calculation section, and thereby determines whether the gradation transformation characteristic curve is within an appropriate range,
wherein the image processing condition calculation section changes the condition and recalculates the gradation transformation characteristic curve, when the determining section has determined that the gradation transformation characteristic curve is not within the appropriate range.

3. The image processing apparatus of claim 2, further comprising:

a region detection section that detects at least one of an irradiation field region as an irradiation field where radiation passes through a subject and a direct irradiation region where radiation that does not pass through the subject is detected, wherein when the image processing condition calculation section recalculates a gradation transformation characteristic curve with a change of the condition, the image processing condition calculation section increases the number of pixels located outside the irradiation field region detected by a region detecting section or inside the direct radiation region detected.

4. The image processing apparatus of claim 1, wherein when the image processing condition calculation section calculates a gradient G such as to be smaller than a gradient obtained by photographing with a screen film system, setting is made such that, with respect to pixels in a predetermined region and difference in pixel value amongst said pixels is greater or equal to 1 before gradation transformation processing, the difference in signal value does not become zero after the gradation transformation processing.

5. The image processing apparatus of claim 1, wherein in the frequency enhancement processing that makes enhancement from a low frequency region in which spatial frequency is lower than 0.5 cycle/mm, the image processing calculation section makes setting such that one of an average, maximum, and minimum values of contrast in a predetermined region is constant.

6. The image processing apparatus of claim 1, further comprising an equalization processing section that performs equalization processing on image data obtained by photographing a subject such that a minimum contrast amplification factor after processing in a predetermined region becomes a predetermined value.

7. The image processing apparatus of claim 2, wherein the determining device determining an appropriate range for said gradation transformation characteristic curve, with respect to pixels in a predetermined region and difference in pixel value amongst said pixels is greater or equal to 1 before gradation transformation processing, the difference in signal value does not become zero after the gradation transformation processing.

8. The image processing apparatus of claim 1, wherein the image processing condition calculation section uses a predetermined fixed value as the gradient G of the gradation transformation characteristic curve.

9. The image processing apparatus of any one of claims 4 to 7, wherein the predetermined region is detected, according to a predetermined reference that is based on one of a predetermined histogram ratio, setting of ROI, and analysis result of a characteristic amount.

10. The image processing apparatus of any one of claims 1 to 7, further comprising an operation section via which input related to setting or change of gradation transformation characteristic curve is made, wherein the image processing condition calculation section determines the gradation transformation characteristic curve, referring to input via the operation section.

**11.** An image processing method that performs gradation transformation processing based on a gradation transformation characteristic curve and frequency enhancement processing based on a frequency enhancement characteristic curve, on image data obtained by radiographic photographing of a subject, comprising:

a step of performing gradation transformation processing on image data obtained by photographing a subject, based on a gradation transformation characteristic curve having a gradient G smaller than a gradient obtained by photographing with a screen film system;

a step of calculating a frequency enhancement characteristic curve for frequency enhancement from a low frequency region in which a spatial frequency is lower than 0.5 cycle/mm, based on the gradation transformation characteristic curve; and

a step of performing frequency enhancement processing on the image data having been subjected to the gradation transformation processing, based on the frequency enhancement characteristic curve calculated by the calculating step.

**12.** The image processing method of claim 11, further comprising:

a step of calculating a standard image processing condition, and its value G and S based on a characteristic amount calculated from image data;

a step of determining whether or not a gradation transformation characteristic curve is within an appropriate range, by comparing the said values G and S with values of G and S of said gradation transformation characteristic curve; and

a step of recalculating the gradation transformation characteristic curve with a change of a condition of calculation, when the determining step has determined that the gradation transformation characteristic curve is not within the appropriate range.

**13.** An image processing program that performs gradation transformation processing based on a gradation transformation characteristic curve and frequency enhancement processing based on a frequency enhancement characteristic curve, on image data obtained by radiographic photographing a subject, and executes on a computer:

processing of gradation transformation on image data obtained by photographing a subject, based on a gradation transformation characteristic curve having a gradient G smaller than a gradient obtained by photographing with a screen film system;

processing of calculation of a frequency enhancement characteristic curve for frequency enhancement from a lower frequency region in which a spatial frequency is lower than 0.5 cycle/mm, based on the gradation characteristic curve; and

processing of frequency enhancement on the image data having been subjected to the gradation transformation processing, based on the frequency enhancement characteristic curve calculated by the calculation processing.

**14.** The image processing program of claim 13, further comprising and executing:

processing of calculating a standard image processing condition and its values for G and S based on a characteristic amount calculated from image data;

processing of determining whether or not the gradation transformation characteristic curve is within an appropriate range, by comparing the said valves for G and S with values for G and S of said gradation transformation characteristic curve; and

processing of recalculating a gradation transformation characteristic curve with a change of a calculating condition, when the determining processing has determined that the gradation transformation characteristic curve is not within the appropriate range.

**Patentansprüche**

**1.** Bildbearbeitungsgerät, das eine Gradationstransformationsbearbeitung und Frequenzverstärungsbearbeitung mit durch radiographisches Photographieren eines Objektes erhaltenen Bilddaten durchführt, folgendes umfassend:

einen Gradationstransformationsbearbeitungsabschnitt, der eine Gradationstransformationsbearbeitung mit durch Photographieren eines Objekts erhaltenen Bilddaten auf der Basis von einer Gradationstransformationseigenschaftskurve mit einem vorbestimmten Bereich für seinen Gradienten G durchführt;

einen Frequenzverstärkungsbearbeitungsabschnitt, der eine Frequenzverstärkungsbearbeitung mit Bilddaten, die der Gradationstransformationsbearbeitung unterzogen wurden, auf der Grundlage einer Frequenzverstärkungseigenschaftskurve, die eine charakteristische der Verstärkungsgrade für die jeweiligen Frequenzen ist, durchführt; und

einen Bildbearbeitungsbedingungsberechnungsabschnitt, der die Gradationstransformationseigenschaftskurve für den Gradationstransformationsbearbeitungsbschnitt und die Frequenzverstärkungseigenschaftskurve für die Frequenzverstärkungseigenschaftskurve für den Frequenzverstärkungsbearbeitungsabschnitt auf der Grundlage der Gradationstransformationseigenschaftskurve berechnet,

wobei der Bildbearbeitungsbedingungsberechnungsabschnitt die Gradationstransformationseigenschaftskurve mit einem Gradienten G, der kleiner ist als ein Gradient, der durch Photographieren mit einem Folienfilmsystem erhalten wurde, und die Frequenzverstärkungseigenschaftskurve für die Frequenzverstärkung aus einer Niederfrequenzregion, in der die Raumfrequenz geringer ist als 0,5 Zyklen/mm, auf der Grundlage der Gradationstransformationseigenschaftskurve berechnet.

2.  Bildbearbeitungsgerät nach Anspruch 1, weiterhin folgendes umfassend:

einen Standardbildbearbeitungszustandsberechnungsabschnitt, der eine Standardbildbildungsbedingung und ihre Werte G und S auf der Grundlage eines charakteristischen, aus Bilddaten berechneten Betrags berechnet; und

einen Bestimmungsabschnitt, der Werte für G und S, berechnet durch den Standardbildbearbeitungsbedingungsberechnungsabschnitt, mit den Werten für G und S der Bearbeitungsbedingungsberechnungsabschnitt mit den Werten für G und S der Gradationstransformationseigenschaftskurve vergleicht, die durch den Bildbearbeitungsbedingungsberechnungsabschnitt berechnet wurde, und **dadurch** bestimmt, ob sich die Gradationstransformationseigenschaftskurve in einem geeigneten Bereich befindet,

wobei der Bildbearbeitungsbedingungsberechnungsabschnitt die Bedingung ändert und die Gradationstransformationseigenschaftskurve erneut berechnet, wenn der Bestimmungsabschnitt bestimmt hat, dass sich die Gradationstransformationseigenschaftskurve nicht in dem geeigneten bereich befindet.

3.  Bildbearbeitungsgerät nach Anspruch 2, weiterhin folgendes umfassend:

einen Regionsnachweisabschnitt, der mindestens eine von einer Bestrahlungsfeldregion als ein Bestrahlungsfeld nachweist, wo Strahlung durch ein Objekt und eine Direktbestrahlungsregion hindurchläuft, wo Strahlung, die nicht durch das Objekt hindurchläuft, nachgewiesen wird, wobei, wenn der Bildbearbeitungsbedingungsberechnungsabschnitt eine Gradationstransformationseigenschaftskurve mit einer Änderung des Zustands neu berechnet, der Bildbearbeitungsbedingungsberechnungsabschnitt die Anzahl von Pixeln erhöht, die außerhalb der Bestrahlungsfeldregion, die durch einen Regionsnachweisabschnitt nachgewiesen wird, oder innerhalb der nachgewiesenen Direktstrahlungsregion liegt.

4.  Bildbearbeitungsgerät nach Anspruch 1, wobei, wenn der Bildbearbeitungsbedingungsberechnungsabschnitt einen Gradienten G so berechnet, dass er kleiner ist als ein Gradient, der durch Photographieren mit einem Folienfilmsystem erhalten wurde, die Einstellung so vorgenommen wird, dass sie bezüglich der Pixel in einer vorbestimmten Region und Differenz im Pixelwert unter den Pixeln vor der Gradationstransformationsbearbeitung größer oder gleich 1 ist, die Differenz im Signalwert nach der Gradationstransformationsbearbeitung nicht Null wird.

5.  Bildbearbeitungsgerät nach Anspruch 1, wobei bei der Frequenzverstärkungsbearbeitung, die die Verstärkung von einer Niederfrequenzregion, in der die Raumfrequenz niedriger ist als 0,5 Cyclen/mm, vornimmt, der Bildbearbeitungsberechnungsabschnitt die Einstellung so vornimmt, dass eines von einem durchschnittlichen Wert, einem maximalen Wert und einem minimalen Wert des Kontrasts in einer vorbestimmten Region konstant ist.

6.  Bildbearbeitungsgerät nach Anspruch 1, weiterhin umfassend einen Egalisierungsbearbeitungsabschnitt, der die Egalisierungsbearbeitung mit Bilddaten durchführt, die durch Photographieren eines Objekts erhalten wurden, derart, dass ein minimaler Kontrastverstärkungsfaktor nach dem Bearbeiten in einer vorbestimmten Region zu einem vorbestimmten Wert wird.

7.  Bildbearbeitungsgerät nach Anspruch 2, wobei die Bestimmungsvorrichtung einen geeigneten Bereich für die Gradationstransformationseigenschaftskurve bezüglich Pixeln in einer vorbestimmten Region bestimmt und die Differenz im Pixelwert unter den Pixeln vor der Gradationstransformationsbearbeitung größer oder gleich 1 ist, die Differenz im Signalwert nach der Gradationstransformationsbearbeitung nicht Null wird.

8. Bildbearbeitungsgerät nach Anspruch 1, wobei der Bildbearbeitungsbedingungberechnungsabschnitt einen vorbestimmten feststehenden Wert als Gradient G der Gradationstransformationseigenschaftskurve verwendet.

9. Bildbearbeitungsgerät nach einem der Ansprüche 4 bis 7, wobei die vorbestimmt Region gemäß einer vorbestimmten Referenz nachgewiesen wird, die auf einem von einem vorbestimmten Histogrammverhältnis, einer ROI-Einstellung und einem Analysewert von einem charakteristischen Betrag beruht.

10. Bildbearbeitungsgerät nach einem der Ansprüche 1 bis 7, weiterhin umfassend einen Arbeitsabschnitt, über den eine Eingabe im Hinblick auf Einstellung oder Änderung der Gradationstransformationseigenschaftskurve vorgenommen wird, wobei der Bildbearbeitungsbedingungberechnungsabschnitt die Gradationstransformationseigenschaftskurve unter Bezugnahme auf die Eingabe über den Arbeitsabschnitt bestimmt.

11. Bildbearbeitungsverfahren, das die Gradationstransformationsbearbeitung auf der Grundlage einer Gradationstransformationseigenschaftskurve und die Frequenzverstärkungsbearbeitung auf der Grundlage einer Frequenzverstärkungseigenschaftskurve mit Bilddaten durchführt, die durch radiographisches Photographieren eines Objekts erhalten werden, folgendes umfassend:

einen Schritt der Durchführung von Gradationstransformationsbearbeitung mit Bilddaten, die durch Photographieren eines Objekts auf der Grundlage einer Gradationstransformationseigenschaftskurve erhalten wurden, mit einem Gradienten G, der kleiner ist als ein Gradient, der durch Photographieren mit einem Folienfilmsystem erhalten wurde;
einen Schritt der Berechnung einer Frequenzverstärkungseigenschaftskurve zur Frequenzverstärkung von einer Niederfrequenzregion, in der eine Raumfrequenz niedriger ist als 0,5 Zyklen/mm, auf der Grundlage der Gradationstransformationseigenschaftskurve; und
einen Schritt der Durchführung von Frequenzverstärkungsbearbeitung mit den Bilddaten, die der Gradationstransformationsbearbeitung unterzogen wurden, auf der Grundlage der durch den Berechnungsschritt berechneten Frequenzverstärkungseigenschaftskurve.

12. Bildbearbeitungsverfahren nach Anspruch 11, weiterhin folgendes umfassend:

einen Schritt der Berechnung einer Standardbildbearbeitungsbedingung und ihrer Werte G und S auf der Grundlage eines aus den Bilddaten berechneten charakteristischen Betrages;
einen Schritt der Bestimmung, ob eine Gradationstransformationseigenschaftskurve innerhalb eines geeigneten Bereichs liegt oder nicht, durch Vergleichen der Werte für G und S mit Werten für G und S der Gradationstransformationseigenschaftskurve; und
einen Schritt der Neuberechnung der Gradationstransformationseigenschaftskurve mit einer Änderung einer Berechnungsbedingung, wenn der Bestimmungsschritt bestimmt hat, dass die Gradationstransformationseigenschaftskurve sich nicht in dem geeigneten Bereich befindet.

13. Bildbearbeitungsprogramm, das eine Gradationstransformationsbearbeitung auf der Grundlage einer Gradationstransformationseigenschaftskurve und eine Frequenzverstärkungsbearbeitung auf der Grundlage einer Frequenzverstärkungseigenschaftskurve mit Bilddaten, die durch radiographisches Photographieren eines Objektes erhalten wurden, und auf einem Computer folgendes durchführt:

Bearbeiten von einer Gradationstransformation mit Bilddaten, die durch Photographieren eines Objekts erhalten wurden, auf der Grundlage einer Gradationstransformationseigenschaftskurve mit einem Gradienten G, der kleiner ist als ein durch Photographieren mit einem Folienfilmsystem erhaltener Gradient;
Bearbeiten von einer Berechnung von einer Frequenzverstärkungseigenschaftskurve aus einer Niederfrequenzregion, in der eine Raumfrequenz niedriger ist als 0,5 Zyklen/mm, auf der Grundlage der Gradationseigenschaftskurve; und
Bearbeiten von einer Frequenzverstärkung mit Bilddaten, die der Gradationstransformationsbearbeitung unterzogen wurden, auf der Grundlage der durch Berechnungsbearbeitung erhaltenen Frequenzverstärkungseigenschaftskurve.

14. Bildbearbeitungsprogramm nach Anspruch 13, weiterhin folgendes umfassend und ausführend:

Bearbeiten von einer Berechnung von einer Standardbildbearbeitungsbedingung und ihrer Werte G und S auf der Grundlage eines aus Bilddaten berechneten charakteristischen Betrags;

Bearbeiten von einer Bestimmung, ob die Gradationstransformationseigenschaftskurve sich in einem geeigneten Bereich befindet oder nicht, durch Vergleichen der Werte für G und S mit Werten für G und S der Gradationstransformationseigenschaftskurve; und

Bearbeiten von einer Neuberechnung einer Gradationstransformationseigenschaftskurve mit einer Änderung einer Berechnungsbedingung, wenn die Bestimmungsbearbeitung bestimmt hat, dass die Gradationstransformationseigenschaftskurve sich nicht in dem geeigneten Bereich befindet.

**Revendications**

1.  Appareil de traitement d'images qui met en oeuvre un traitement de transformation de gradation et un traitement d'amplification de fréquence avec des données d'images obtenues par photographie radiographique d'un sujet, comprenant :

    une partie de traitement de transformation de gradation qui met en oeuvre un traitement de transformation de gradation avec des données d'images obtenues par photographie d'un sujet, à base d'une courbe de caractéristique de transformation de gradation ayant une gamme prédéterminée pour le gradient de celle-ci ;
    une partie de traitement d'amplification de fréquence qui met en oeuvre un traitement d'amplification de fréquence avec des données d'images qui ont été soumises à un traitement de transformation de gradation à base d'une courbe de caractéristique d'amplification de fréquence étant une courbe de caractéristique des degrés d'amplification pour les fréquences respectives ; et
    une partie de calcul de condition de traitement d'image qui calcule la courbe de caractéristique de transformation de gradation pour ladite partie de traitement de transformation de gradation et la courbe de caractéristique d'amplification de fréquence pour ladite partie de traitement d'amplification de fréquence, à base de la courbe de caractéristique de transformation de gradation,
    dans lequel la partie de calcul de condition de traitement d'image calcule ladite courbe de caractéristique de transformation de gradation ayant un gradient G inférieur à un gradient obtenu par photographie avec un système de film écran, et calcule ladite courbe de caractéristique d'amplification de fréquence pour amplification de fréquence à partir d'une région de basse fréquence dans laquelle la fréquence spatiale est inferieure à 0,5 cycle/mm, à base de ladite courbe de caractéristique de transformation de gradation.

2.  Appareil de traitement d'image selon la revendication 1, comprenant en outre :

    une partie de calcul de condition de traitement d'image standard qui calcule une condition formant d'image standard et les valeurs G et S de celle-ci à base d'un nombre caractéristique calculé à partir des données d'image ; et
    une partie de détermination comparant lesdites valeurs pour G er S calculées par la partie de calcul de condition de traitement d'image standard aux valeurs pour G et S de ladite courbe de caractéristique de transformation de gradation calculée par la partie de calcul de condition de traitement d'image et ainsi déterminer si la courbe de caractéristique de transformation de gradation est dans la gamme appropriée,
    dans lequel la partie de calcul de condition de traitement d'image modifie la condition et recalcule la courbe caractéristique de transformation de gradation, lorsque la partie de détermination a déterminé que la courbe caractéristique de transformation de gradation n'est pas dans la gamme appropriée.

3.  Appareil de traitement d'image selon la revendication 2, comprenant en outre :

    une partie de détermination de région qui détecte au moins l'une d'une région de champs d'irradiation dans lequel la radiation passe à travers un sujet et une région d'irradiation directe dans laquelle la radiation qui ne passe pas à travers le sujet est détectée, dans lequel lorsque la partie de calcul de condition de traitement d'image recalcule une courbe de caractéristique de transformation de gradation ayant une modification de la condition, la partie de calcul de condition de traitement d'image fait augmenter le nombre de pixels localisés en dehors de la région de champs d'irradiation détectée par une partie de détection de région ou au sein de la région d'irradiation directe détectée.

4.  Appareil de traitement d'image selon la revendication 1, dans lequel lorsque la partie de calcul de condition de traitement d'image calcule un gradient G tel qu'il est inférieur à un gradient obtenu par photographie avec un système de film écran, le réglage est fait tel qu'il est, relatif aux pixels dans une région prédéterminée et à la différence dans la valeur de pixel parmi lesdits pixels, supérieur ou égal à 1 avant le traitement de transformation de gradation, la différence dans la valeur de signal ne devient pas zéro âpres le traitement de transformation de gradation.

5.  Appareil de traitement d'image selon la revendication 1, dans lequel dans le traitement d'amplification de fréquence faisant l'amplification d'une région de basse fréquence dans laquelle la fréquence spatiale est inferieure à 0,5 cycle/mm, la partie de calcul de traitement d'image fait le réglage tel que l'une des valeurs de contraste moyennes, maximales et minimales dans une région prédéterminée est constante.

6.  Appareil de traitement d'image selon la revendication 1 comprenant en outre une partie de traitement d'égalisation qui met en oeuvre le traitement d'egalisation avec des données d'image obtenues par photographie d'un sujet tel qu'un facteur d'amplification de contraste minimal après le traitement dans une région prédéterminée devient une valeur prédéterminée.

7.  Appareil de traitement d'image selon la revendication 2, dans lequel le dispositif de détermination déterminant une gamme appropriée pour ladite courbe caractéristique de transformation de gradation en relation aux pixels dans une région prédéterminée et la différence dans la valeur de pixel parmi lesdits pixels est supérieure ou égale à 1 avant le traitement de transformation de gradation, la différence dans la valeur de signal ne devient pas zéro après le traitement de transformation de gradation.

8.  Appareil de traitement d'image selon la revendication 1, dans lequel la partie de calcul de condition de traitement d'image utilise une valeur fixe prédéterminée en tant que le gradient G de la courbe caractéristique de transformation de gradation.

9.  Appareil de traitement d'image selon l'une des revendications 4 à 7, dans lequel la région prédéterminée est détectée, selon une référence prédéterminée qui est basée sur un rapport d'histogramme prédéterminé, réglage de ROI et résultat d'analyse d'un nombre caractéristique.

10. Appareil de traitement d'image selon l'une des revendications 1 à 7, comprenant en outre une partie d'opération par laquelle la saisie liés au réglage ou modification de la courbe de caractéristique de transformation de gradation est fait, dans lequel la partie de calcul de condition de traitement d'image détermine la courbe caractéristique de transformation de gradation se rapportant à la saisie par la partie d'opération.

11. Procédé de traitement d'image qui met en oeuvre un traitement de transformation de gradation basé sur une courbe de caractéristique de transformation de gradation et un traitement d'amplification de fréquence basé sur une courbe de caractéristique d'amplification de fréquence avec des données d'image obtenues par photographie radiographique d'un sujet, comprenant :

    une étape de réalisation un traitement de transformation de gradation avec des données d'image obtenues par photographie d'un sujet, basé sur une courbe de caractéristique de transformation de gradation ayant un gradient G inférieur à un gradient obtenu par photographie avec un système de film écran,
    une étape de calcule une courbe de caractéristique d'amplification de fréquence pour l'amplification de fréquence d'une région de basse fréquence dans laquelle une fréquence spatiale est inferieure à 0,5 cycle/mm, à base de ladite courbe de caractéristique de transformation de gradation ; et
    une étape de réalisation le traitement d'amplification de fréquence avec les données d'images qui ont été soumises à un traitement de transformation de gradation à base de la courbe de caractéristique d'amplification de fréquence calculé par l'étape de calcul.

12. Procédé de traitement d'image selon la revendication 11, comprenant en outre :

    une étape de calcule d'une condition standard de traitement d'image et des valeurs G et S de celle-ci basé sur le nombre caractéristique calculé à partir des données d'image ;
    une étape de déterminer si une courbe de caractéristique de transformation de gradation est dans une gamme appropriée ou non, par comparaison les valeurs pour G et S aux valeurs pour G et S de ladite courbe de caractéristique de transformation de gradation ; et
    une étape de recalculer la courbe de caractéristique de transformation de gradation ayant une modification d'une condition de calcul lorsque l'étape de détermination a déterminée que la courbe de caractéristique de transformation de gradation n'est pas dans la gamme appropriée.

13. Program de traitement d'image qui met en oeuvre un traitement de transformation de gradation basé sur une courbe de caractéristique de transformation de gradation et un traitement d'amplification de fréquence basé sur une courbe de caractéristique d'amplification de fréquence avec des données d'image obtenues par photographie radiographique d'un sujet, et exécute sur un calculateur les é'tapes suivantes :

traiter la transformation de gradation avec des données d'image obtenues par photographie d'un sujet basé sur une courbe de caractéristique de transformation de gradation ayant un gradient G inférieur à un gradient obtenu par photographie avec un système de film écran ;

traiter le calcul d'une courbe de caractéristique d'amplification de fréquence d'une région de fréquence pour l'augmentation de fréquence d'une région de fréquence plus basse dans laquelle une fréquence spatiale est inferieure à 0,5 cycle/mm, à base de ladite courbe de caractéristique de gradation ; et

traiter l'amplification de fréquence avec des données d'image qui ont été soumises à un traitement de transformation de gradation à base d'une courbe de caractéristique d'amplification de fréquence calculée par le traitement de calcul.

**14.** Program de traitement d'image selon la revendication 13, comprenant et exécutant en outre :

traiter le calcul d'une condition standard de traitement d'image et les valeurs G et S de celle-ci basé sur un nombre caractéristique calculé à partir des données d'image ;

traiter la détermination si la courbe de caractéristique de transformation de gradation est dans une gamme appropriée ou non par comparer lesdites valeurs pour G et S avec des valeurs pour G et S de ladite courbe de caractéristique de transformation de gradation ; et

traiter le recalcule d'une courbe de caractéristique de transformation de gradation avec une modification d'une condition de calcul lorsque le traitement de la détermination a déterminé que la courbe de caractéristique de transformation de gradation n'est pas dans la gamme appropriée.

# FIG. 1

160 IMAGE PROCESSING SECTION

IMAGE DATA →

110
IMAGE DATA INPUT SECTION

161
EQUALIZATION PROCESSING SECTION

162
GRADATION TRANSFORMATION PROCESSING SECTION

163
FREQUENCY ENHANCEMENT PROCESSING SECTION

180
IMAGE OUTPUT SECTION

→ IMAGE DATA HAVING BEEN SUBJECTED TO IMAGE PROCESSING

120
STANDARD IMAGE PROCESSING CONDITION CALCULATION SECTION

140
IMAGE PROCESSING CONDITION DETERMINING SECTION

IMAGE PROCESSING CONDITION CALCULATION SECTION
130

EP 1 820 447 B1

22

## FIG. 2

```
            ┌─────────┐
            │  START  │
            └────┬────┘
                 ▼
    ┌──────────────────────┐
    │   INPUT IMAGE DATA    │ S1
    └──────────┬───────────┘
               │                      ┌──────────────────────────┐
               ├─────────────────────▶│ EXTRACT CHARACTERISTIC    │ S4
               ▼                      │        AMOUNT A           │
    ┌──────────────────────┐          └──────────┬───────────────┘
    │    DETERMINE ROI      │ S2                  ▼
    └──────────┬───────────┘          ┌──────────────────────────┐
               │                      │  DETERMINE (S, G)' FROM   │ S5
               ▼                      │  CHARACTERISTIC  AMOUNT A │
    ┌──────────────────────┐          └──────────┬───────────────┘
    │ DETERMINE (S, G) FROM ROI │ S3             │
    └──────────┬───────────┘                     │
               ▼                                 │
    ┌──────────────────────┐                     │
    │   COMPARE (S, G) FROM │                     │
    │  ROI WITH (S, G)' FROM │ S6                 │
    │ CHARACTERISTIC AMOUNT A│◀───────────────────┘
    └──────────┬───────────┘
               ▼            S7
          ◇──────────────◇       Y
         ◇ WITHIN PREDETERMINED ◇─────────┐
          ◇    RANGE?    ◇                 │
               │ N                         │
               ▼                           │
    ┌──────────────────────┐               │
    │     CHANGE ROI        │ S8            │
    └──────────────────────┘               │
               │                           │
               ▼                           │
    ┌──────────────────────┐               │
    │   DETERMINE (S, G)    │ S9 ◀──────────┘
    └──────────┬───────────┘
               ▼
    ┌──────────────────────┐
    │ DETERMINE FREQUENCY   │
    │   ENHANCEMENT         │ S10
    │  CHARACTERISTIC       │
    │ CORRESPONDING TO (S, G)│
    └──────────┬───────────┘
               ▼
    ┌──────────────────────┐
    │ PERFORM GRADATION     │
    │  TRANSFORMATION       │ S11
    │PROCESSING AND FREQUENCY│
    │ ENHANCEMENT PROCESSING │
    └──────────┬───────────┘
               ▼
            ┌─────────┐
            │   END   │
            └─────────┘
```

23

# FIG. 3 ( a )

GRADATION TRANSFORMATION CHARACTERISTIC

# FIG. 3 ( b )

FREQUENCY ENHANCEMENT CHARACTERISTIC

# FIG. 4

EP 1 820 447 B1

FIG. 5

100 IMAGE PROCESSING APPARATUS

160 IMAGE PROCESSING SECTION

161 162 163

EQUALIZATION PROCESSING SECTION

GRADATION TRANSFORMATION PROCESSING SECTION

FREQUENCY ENHANCEMENT PROCESSING SECTION

110

IMAGE DATA → IMAGE DATA INPUT SECTION

180

IMAGE OUTPUT SECTION → IMAGE DATA HAVING BEEN SUBJECTED TO IMAGE PROCESSING

120

STANDARD IMAGE PROCESSING CONDITION CALCULATION SECTION

140

IMAGE PROCESSING CONDITION DETERMINING SECTION

115

OPERATION SECTION

IMAGE PROCESSING CONDITION CALCULATION SECTION

130

EP 1 820 447 B1

# FIG. 6 (a)  FIG. 6 (c)

# FIG. 6 (b)

# FIG. 7 (a)

GRADATION
TRANSFORMATION
CHARACTERISTIC

SIGNAL VALUE

LIGHT EMISSION VALUE

READING CHARACTERISTIC

VALUE S

VALUE G

VALUE G

SH
IMAGE DATA

SL

L1 L2

log (X-RAY DOSE)

# FIG. 7 (b)

FREQUENCY

REFERENCE VALUE L          REFERENCE VALUE H

SIGNAL VALUE

# FIG. 7 (c)

FREQUENCY

SL                    SH

SIGNAL VALUE

## FIG. 8

LEVEL

T2

T1

D2

D1

CC

log (RADIATION DOSE)

## FIG. 9

OUTPUT IMAGE DATA

T2'

T1'

T1          T2

NORMALIZED IMAGE DATA

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H57578 A
- JP S6226047 B, Tokkaisho
- JP H0916762 B
- JP 2002133410 A
- JP 2001120524 A
- US 20010038707 A

- JP H11142998 B
- JP 2002156716 A
- JP H6342098 B
- JP H990048 B
- JP 2005109867 A